Europäisches Patentamt

European Patent Office

Office européen des brevets

(11) Publication number: **0 297 290**
**A2**

## EUROPEAN PATENT APPLICATION

(21) Application number: 88108601.1

(51) Int. Cl.⁴: **G01N 33/52 , G01N 33/553**

(22) Date of filing: 30.05.88

(30) Priority: 10.06.87 US 60745

(43) Date of publication of application:
04.01.89 Bulletin 89/01

(84) Designated Contracting States:
AT BE CH DE ES FR GB IT LI LU SE

(71) Applicant: MILES INC.
1127 Myrtle Street
Elkhart Indiana 46514(US)

(72) Inventor: Morris, David L.
23274 Ronda Drive
Elkhart, IN 46514(US)
Inventor: Motsenbocker, Marvin
10180 Viking Drive
Eden Prairie, MN 55 344(US)

(74) Representative: Dänner, Klaus, Dr. et al
c/o Bayer AG Konzernverwaltung RP
Patentabteilung
D-5090 Leverkusen 1 Bayerwerk(DE)

(54) Method for magnetically separating labeled reagent in an immunometric binding assay.

(57) A method for the magnetic separation of the free species of a labeled reagent in an immunometric binding assay involving binding reactions in a liquid reaction mixture among analyte from a liquid test medium, a magnetically responsive reagent particle comprising the analyte or a binding analog thereof immobilized thereto, and a labeled reagent comprising a specific binding partner for the analyte labeled with a detectable chemical group. Analyte from the test medium binds to the labeled reagent and any of the labeled reagent not so bound binds to the magnetically responsive reagent particle which are separated on a chromatographic separation and detection test device comprising a separation zone and a detection zone for receiving the analyte bound to the labeled reagent. The reaction mixture and a magnetic field are applied to the separation zone whereby the magnetically responsive reagent particle is magnetically retained at the separation zone and the analyte bound to the labeled reagent is transported into the detection zone where the detectable chemical group thereof provides a detectable signal which is measured and correlated to the amount of analyte in the liquid test medium.

EP 0 297 290 A2

# METHOD FOR MAGNETICALLY SEPARATING LABELED REAGENT IN AN IMMUNOMETRIC BINDING ASSAY

## BACKGROUND OF THE INVENTION

The present invention relates to specific binding assays which require the separation of free and bound species of a labeled reagent for the determination of an analyte in a liquid test medium. In particular, the present invention relates to heterogeneous immunoassays employing reagent particles for the separation of the free species of a labeled reagent from the bound species of a labeled reagent.

Various heterogeneous specific binding assays have been developed which generally involve specific binding interactions in a reaction mixture between the analyte to be detected, a specific binding partner for the analyte, and a labeled reagent which can be the same or different as the binding partner for the analyte. When performing such assays, the labeled reagent becomes bound to its corresponding binding partner to generate a bound species of the labeled reagent, any of the labeled reagent not so bound being the free species, wherein the extent of binding is a function of the amount of analyte present. However, since the detectable response of the labeled reagent is essentially indistinguishable in the bound and the free species, it is therefore necessary to physically separate such bound and free species from each other in order to effectively determine the amount of analyte present. Once the bound and free species of the labeled reagent have been separated from each other, the amount of label present in either fraction is determined by measuring the activity of the particular label of the labeled reagent and correlating such activity to the amount of analyte present.

Typically, such separation is accomplished by employing particulate solid phase reagent materials which are capable of binding to the free species in order to physically separate the free species from the bound species, but which nevertheless require cumbersome and often inconvenient manipulative steps to effect such separation. For example, U.S. Patent No. 4,200,436 describes an immunoassay employing an immobilized form of an antigen to be measured which is added to an immunoassay reaction mixture for binding to the free species of the labeled reagent. However, in order to separate the free species bound to the immobilized antigen from the bound species, the reaction mixture must be centrifuged to effect such separation. Furthermore, once the reaction mixture has been centrifuged, either an aliquot must be obtained from the supernatant, or the supernatant must be removed, in order to measure the label of the bound species contained in the supernatant, or the label of the sedimented free species, respectively.

Although various methods have been described which do not require a centrifugation step, such methods nevertheless still require cumbersome manipulative steps in order to measure the label in either the free species or the bound species of the labeled reagent. For example, U.S. Patent No. 4,628,036 describes liquid binding assays performed in, for example, a test tube, employing magnetic particles to which an antigen or antibody is immobilized and capable of binding to the free species of a labeled reagent as described above. The free species bound to the magnetic particles are magnetically separated with, for example, a permanent magnet held at the bottom of the test tube to attract and thereby separate the bound particules. Although a centrifugation step is not required to effect the separation of the free species from the bound species, it is nevertheless necessary to obtain an aliquot of the supernatant or to remove the supernatant in order to measure the label in one of the species as described above.

Furthermore, various methods have been described employing a chromatographic test device which, together with the use of solid phase reagent materials, effect the separation of the bound and free species of a labeled reagent without a centrifugation step. For example, European Patent Application Publication No. 120,602 describes an assay system employing a labeled reagent present partly in a form which is mobile on a selected chromatographic medium and partly in a form which is immobile. An antibody to the analyte is attached or immobilized to a particulate solid phase material which is immobile on the selected chromatographic medium and which binds to sample analyte. A labeled form of the antibody to the analyte is employed which binds to vacant binding sites of the sample analyte bound to the immobilized antibody. Any of the labeled antibody not so bound is caused to migrate away from the point of application on the chromatographic medium by applying a solvent thereto whereby the amount of label of the immobile labeled antibody bound to the solid phase material of the free labeled antibody is measured and related to the amount of analyte in the sample.

European Patent Application Publication No. 120,602 further describes the separation of agglutinated particles from nonagglutinated particles in an assay system employing latex particles as the particulate solid phase material. Depending upon the particular immunoassay format followed, e.g., competitive immunoassay or sandwich immunoassay, the latex particules agglutinate in the absence or presence, respectively, of analyte, whereby the agglutinated particles are separated from nonagglutinated particles on a selected chromatographic medium on which the former are immobile and the latter are mobile, and the amount of label of the labeled reagent of the one or the other is measured.

The concentration of agglutinated particles to be measured on a bibulous support is also described by European Patent Application Publication No. 135,324 which provides an observable signal in relation to the concentration of particles on the support. In the presence of analyte, the particles employed can be inhibited from migrating or permitted to migrate, and the presence of a detectable signal at a localized or concentrated site on the support can be related to the presence of analyte. The concentration of particles is achieved by providing specific binding members as bridges between the particles. For example, in an assay for a hapten, the hapten is coupled to colored beads, which, individually, are mobile on the support, and immobile when agglutinated. The sample hapten is reacted with antibodies thereto, and then reacted with the beads for binding to available binding sites to the hapten on the beads wherein, for example, in the absence of sample hapten, there is a substantial amount of bridging between the beads by the antibodies, i.e., agglutination. Upon contact with the support, a concentration of agglutinated beads, indicated by the color of the beads, is observed in the absence of hapten as a result of the immobility thereof on the support, whereas in the presence of hapten, there is no agglutination and therefore no concentration of beads and no observable color. Similarly, for the determination of antigen, beads labeled with antibodies to the antigen and with enzymes are incubated with the sample antigen wherein bridges are formed between the beads by the antigen binding to the antibodies of the beads. The presence of antigen results in agglutination of the beads as heretofore described, and the support is contacted with a substrate for the enzyme and an appropriate indicator compound to indicate the presence of the enzyme bound to the antigen-agglutinated beads retained by the support at the site of concentration.

A method for the detection and determination of the concentration of sample analyte by agglutination or agglutination inhibition is described by German Offenlegungsschrift No. 3,511,012. The method employs a particle comprising at least one component of a bioaffinity binding system, e.g.,

antigen or antibody, and at least one component of a signal producing system, e.g., an enzyme, which are used in an agglutination or agglutination inhibition assay as heretofore described and as known in the art. The separation of agglutinated and nonagglutinated particles is carried out on a test device comprising an application zone, a reaction zone, a separation zone and a detection zone. The particles are either incorporated into the reaction zone or applied thereto prior to or simultaneously with the application of the sample antibody or antigen to the application zone. The presence of sample antibody or antigen either produces or prevents agglutination of the particles, respectively. The separation is accomplished by the separation zone which is permeable to the nonagglutinated particles but impermeable to the agglutinated particles. The detection zone is incorporated with another component of the signal producing system, e.g., a substrate for the enzyme, whereby the enzyme label of the nonagglutinated particles which migrate therein react with the substrate to provide a signal which is correlated to the amount of sample antigen or antibody.

One of the disadvantages of performing such competitive and two-site sandwich immunoassays where the labeled reagent bound to the solid phase material is measured, is the necessity to remove any of the labeled reagent not so bound which, if present, would otherwise result in an interfering background signal and reduced assay sensitivity. Accordingly, when performing such immunoassays employing a test device for the separation of the bound and free species of a labeled reagent, an additional wash step, employing a wash solution, is necessary to cause any of the mobile labeled reagent to migrate away from the site of the immobile labeled reagent. Furthermore, where an enzyme is employed as the label of the labeled reagent, a soluble enzyme substrate incorporated into the device would be solubilized and washed away by the wash solution and, accordingly, would therefore require either an additional step of applying an enzyme substrate solution subsequent to the wash solution, or incorporating the enzyme substrate into the wash solution.

Where the mobile species of the labeled reagent is instead measured according to such competitive and sandwich immunoassay formats, the precise measurement thereof requires that the amount of labeled reagent employed in the immunoassay be carefully controlled. Furthermore, such excess amount is limited by the need to have a signal in a measureable range. Such measurement will also result in a dose response having a high background signal in the absence of analyte.

Furthermore, in a competitive immunoassay format, the amount of antibody immobilized to the solid phase material is also critical to the performance of the immunoassay, the dose response is not linear over a wide range of analyte concentration and difficult to linearize for a narrow range, and requires extended periods of incubation.

Still further, such methods which depend upon the agglutination of particles for the separation of the bound and free species of a labeled reagent required aggregates of only a few particles to be separated from monodisperse particles, where the difference in size thereof is one order of magnitude or less. Accordingly, the choice of the chromatographic matrix must be carefully made in order to distinguish agglutinated particles from nonagglutinated particles. Such methods also require that the particles remain monodisperse prior to performance of the particular assay, as well as during the performance of an immunoassay in the absence of analyte. In particular, the agglutination of such particles can nevertheless occur, for example, during periods of storage or in the presence of biological fluids, respectively.

Although European Patent Application Publication No. 120,602 suggests the use of magnetically susceptable forms of the labeled antibody which can be rendered immobile in the test device described therein with a magnet field, the aforementioned disadvantages will nevertheless be encountered, regardless of the manner by which the particular solid phase material is prevented from migrating therethrough.

Accordingly, it is an object of the present invention to provide a convenient method for the rapid separation and detection of the bound species of a labeled reagent on a chromatographic test device which does not require a wash step or other cumbersome manipulative steps as heretofore described.

It is a further object of the present invention to provide an efficient method for separating the bound species of a labeled reagent into distinct, measurable species thereof.

Another object of the present invention is to provide a method for the specific binding assay determination of analyte on a chromatographic test device which does not require additional reagents or developer solutions to provide a detectable signal thereon.

Still another object of the present invention is to provide a method for the specific binding assay determination of analyte on a chromatographic test device which provides a detectable signal which results in a linear dose response with little or no interfering background signal.

## SUMMARY OF THE INVENTION

The present invention provides a method for the magnetic separation of the bound and free species of a labeled reagent in an immunometric binding assay employing a magnetically responsive reagent particle comprising the analyte or a binding analog thereof immobilized to an insoluble, paramagnetic particle, and a labeled reagent comprising a specific binding partner for the analyte labeled with a detectable chemical group. The liquid test medium containing the analyte, the labeled reagent, and the magnetically responsive reagent particle are simultaneously or sequentially combined, preferably sequentially, to form an aqueous liquid reaction mixture whereby the labeled reagent binds to the analyte to form the bound species thereof, and the magnetically responsive reagent particle is present in an amount in excess of that which is capable of binding to substantially all of the free species of the labeled reagent which does not bind to the analyte.

The free species of the labeled reagent bound to the magnetically responsive reagent particle is magnetically separated from the bound species on a chromatographic separation and detection test device comprising a solid, porous matrix which is capable of transporting aqeuous liquids by capillary action and which comprises a separation zone and a detection zone which is situated a predetermined distance away from and in liquid transport contact with the separation zone. The separation and detection zones are physically distinct portions which can be located along the length of an elongate strip composed of a single, continuous, solid, porous matrix material, or the physically distinct portions comprising the separation and detection zones can be composed of physically distinct solid, porous matrix materials located along the length of the elongate strip.

In carrying out the method of the present invention, an aliquot of the reaction mixture is applied to the separation zone of the test device and a magnetic field applied thereto, whereby the magnetically responsive reagent particle having the free species of the labeled reagent immobilized thereto is magnetically retained and thereby prevented from migrating out of the separation zone. Since the solid, porous matrix provides a chromatographic medium for the unhindered migration of the bound species therethrough, the bound species of the labeled reagent is transported by the liquid test medium from the separation zone into the detection zone. The detectable chemical group of the bound species in the detection zone is then measured and correlated to the amount of analyte present in the test medium.

Since the method of the present invention does not require a wash step to cause the bound species to migrate from the separation zone into the detection zone, additional steps are not required to effect the separation thereof once the aliquot of the reaction mixture is applied to the separation zone.

Furthermore, the separation of the bound species from the immobilized free species of the labeled reagent commences essentially simultaneously with the initial generation of the detectable signal in the detection zone without additional manipulative or mechanical steps. Accordingly, the controlled separation and detection of the bound species of the labeled reagent during a substantially short period of time is provided.

Still further, since the immunometric assay method employed according to the present invention does not require a wash step to remove excess and/or nonspecifically bound labeled reagent, a soluble form of a detectant component for the label of the labeled reagent, such as a substrate for an enzyme label, can be incorporated into the test device.

## BRIEF DESCRIPTION OF THE DRAWINGS

Fig. 1 is a perspective view of a magnet positioned under a test device according to the present invention comprising a separation zone and a detection zone located at physically distinct portions along the length of an elongate strip.

Fig. 2 is a perspective view of a magnet positioned under a test device according to the present invention comprising a separation zone and a detection zone composed of physically distinct matrix materials in liquid transport contact located along the length of an elongate strip.

## DESCRIPTION OF THE PREFERRED EMBODIMENTS

The magnetic separation of the bound and free species of a labeled reagent on a chromatographic test device according to the present invention provides a convenient method for the immunometric determination of analyte in a liquid test medium without the need for cumbersome manipulative steps, such as centrifugation, sedimentation, decantation, and the like. In carrying out the method of the present invention, the aqueous liquid reaction mixture is first formed comprising the liquid test medium, the labeled reagent, and the magnetically responsive reagent particle. The reaction mixture is then applied to the separation zone of the separation and detection test device and a magnetic field applied thereto. Accordingly, the magnetically responsive reagent particle having the free species of the labeled reagent immobilized thereto is magnetically retained at the separation zone and prevented from migrating into the detection zone while, at the same time, permits the capillary transport of the bound species out of the separation zone and into the detection zone to effect such separation.

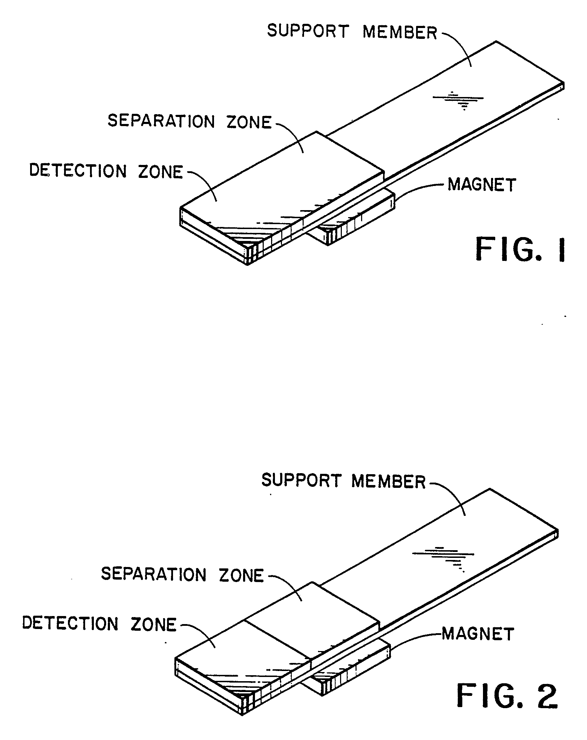

The magnetic field can be applied to the separation zone according to methods known in the art, such as with a permanent magnet or electrically induced with an electromagnet positioned beneath the separation zone (see Figs. 1 & 2). Preferably, the magnetic field is applied prior to or simultaneously with the application of the reaction mixture to the separation zone in order to prevent any substantial migration of the magnetically responsive reagent particle upon contact therewith. It is to be understood that although the magnetic field can be applied subsequent to the application of the reaction mixture to the separation zone as well, the separation zone matrix must then be capable of preventing the substantial migration of the magnetically responsive reagent particle prior to the application of the magnetic field thereto.

According to the present invention, the magnetically responsive reagent particle is present in an amount in excess of that which is capable of binding to all of the free species of the labeled reagent which does not become bound to the analyte from the test medium. Accordingly, the excess amount of the magnetically responsive reagent particle is at an excess amount such that a sufficient amount is present to bind substantially all of the free species of the labeled reagent for the ultimate immobilization and separation of substantially all of the free species from the bound species.

Preferably, the labeled reagent is also present in an excess amount relative to the estimated amount of concentration of suspected analyte in the test medium. Such excess amount provides a sufficient number of specific binding partners for the suspected analyte in the test medium so that substantially all of the suspected analyte becomes bound by the labeled reagent. It is to be appreciated that since the excess labeled reagent binds and thereby renders substantially all of the suspected analyte in the test medium detectable, in accurate and highly sensitive determination of the analyte can be made.

The use of excess amounts of reagents as heretofore described results in favorable kinetic and/or equilibrium advantages. In particular, excess amounts of reagents accelerate the kinetics of the binding reactions and shifts the equilibrium of the reaction to favor the formation of the bound species, even at low analyte concentrations, whereby the limiting reagent of the assay becomes the analyte itself. Accordingly, since the assay reagents are in excess amounts, the effect of variations in the concentration thereof on assay performance is less significant than, for example, that which is encountered in a conventional competitive immunoassay format, and a margin of error in reagent additions is thereby permitted when made at least in excess amounts.

Furthermore, by shifting the equilibrium as heretofore described, it also becomes possible to maximize the amount of analyte bound by the labeled reagent and therefore optimize the sensitivity of the assay. Still further, where the single antibody immunometric method of the present invention is employed, the presence of nonspecific binding does not create a background signal which would reduce the sensitivity of the assay, such as that encountered in conventional sandwich immunoassays where two antibody binding reactions are involved and wherein the label bound to a solid phase material or reagent particle is measured. The single antibody immunometric method also permits the application thereof to the detection of analytes having a single epitope, which is not feasible in a sandwich immunoassay. Furthermore, where monoclonal monovalent antibodies are employed in excess amounts in an assay, together with excess amounts of other assay reagents, the production of a linear dose response curve which facilitates the application of such assay format to a convenient calibration procedure is provided.

It is to be understood that although the aforementioned assay reagents can be combined simultaneously to form the reaction mixture as heretofore described, the assay reagents are preferably combined sequentially to form the reaction mixture. In particular, a first rection mixture comprising the test medium containing the analyte and the labeled reagent is formed and incubated for a predetermined period of time sufficient to permit binding of all of the analyte from the test medium to the labeled reagent. The first reaction mixture is then combined with the magnetically responsive reagent particles to form a second reaction mixture and incubated for a predetermined period of time to permit binding of all of the free species of the labeled reagent to the magnetically responsive reagent particles.

## Reagent Particle

The magnetically responsive reagent particle employed by the method of the present invention comprises a water insoluble magnetically responsive particulate material which responds to a magnetic field without resultant permanent magnetization, hereinafter referred to as "paramagnetic" or "paramagnetism". For example, such paramagnetic behavior is typically exhibited by iron oxides having a crystal size less than about 300 Å, whereas iron oxides having a crystal size greater than about 500 Å are characterized by responsiveness to a magnetic field with resultant permanent magnetization. Accordingly, it is to be understood that the advantage of employing magnetically responsive reagent particles comprising a paramagnetic particulate material as previously described is that they can be exposed to magnetic fields, without becoming permanently magnetized, which would otherwise result in the undesirable magnetic aggregation thereof during the performance of an immunoassay.

Generally, such magnetically responsive reagent particles comprise any paramagnetic material having a crystal size less than about 300 Å and to which the analyte under determination, or a binding analog thereof, is capable of being covalently bound, noncovalently bound (e.g., physical absorption), or otherwise immobilized thereto. Such paramagnetic materials include, but are not intended to be limited to, transition metals such as iron, nickel, magnesium, manganese, cobalt, zinc, copper, titanium, and the like; transition metal oxides and transition metal salts thereof, such as $Fe_3O_4$ and ferrite and $FeCl_2$, $FeCl_3$ and $CoCl_2$, respectively; and transition metal alloys such as Ni-Nio, Ni-$TiO_4$, and Mn-Zn.

Although the analyte or binding analog thereof can be immobilized to such paramagnetic materials, the magnetically responsive reagent particle is preferably a magnetically responsive particle comprising a core of such paramagnetic material surrounded by an absorptively or covalently bound outer shell or coating, the outer shell or coating can be selected from a wide variety of materials known in the art which are suitable coating materials for paramagnetic material cores and which are capable of being coupled to the analyte under determination or binding analogs thereof. For example, silane, which is a silicon-functional silicon compound characterized in that the silicon portion of the molecule has an affinity for inorganic materials and the organo functionalities thereof are capable of being coupled to bioaffinity adsorbents, can be employed for such purpose. Other outer shell or coating materials include, but are not intended to be limited to polystyrene, polyacrylates, polyacrylamide, or naturally occurring materials such as polysaccharides, particularly cross-linked polysaccharides, such as agarose, dextran, microcrystalline cellulose, starch, polyvinyltoluene, or styrenebutadiamine copolymers, polyacrolein microspheres, polyurethane, pollen particles, sporopollenin, polystyrene or polyvinylnapthalene cores surrounded by shells of polyclycidyl methacrylate, microcrystalline cellulose or combinations thereof, polyvinyl alcohol, copolymers of hydroxyethyl methacrylate and methyl methacrylate, silicones and silica, glass, rubber, nylon, diatomaceous earth, silica, and the like.

Such magnetically responsive particles are commercially available, or can be prepared according to methods known in the art, such as described by U.S. Patent No. 4,335,094 which employs a polymer having lattices or pores with a magnetic core surrounded by a vinylaromatic polymer; U.S. Patent No. 4,452,773 which employs a colloidal magnetic iron oxide coated with a polysaccharide having pendant functional groups for covalently binding biological molecules thereto; and U.S. Patent Nos. 4,554,088 and 4,628,037 which employ a metal oxide core generally surrounded by a silane coating.

Generally, the largest dimension of the magnetically responsive reagent particles employed according to the present invention is less than 50 microns, preferably from between about 0.1 microns and 20 microns, more preferably from between about 0.5 microns and 3.0 microns. In particular, the magnetically responsive reagent particles are preferably uniform latex particles which are dispersable or suspendable in aqueous media without significant gravitational settling and therefore capable of remaining in suspension in the reaction mixture without constant mixing, i.e., water suspensible. Accordingly, together with Brownian motion and the high surface to volume ratio, efficient and rapid binding kinetics are assured.

Although the magnetically responsive reagent particles are generally in the form of a bead, other configurations may be employed as well which would be compatible with the teachings of the present invention. For example, such configurations include rods, fibers, geometric configurations, and the like, and irregular configurations thereof. Furthermore, where it is desirable to covalently bind the analyte or binding analog thereof to the magnetically responsive reagent particle, the particle should be polyfunctional or capable of being polyfunctionalized with functional groups which, for example, can be incorporated according to covalent coupling techniques known in the art [see, for example, Cuatrecasas, J. Biol. Chem. Vol. 245, p. 3059(1970)]. Functional groups include carboxylic acids, aldehydes, amines, amides, activated ethylenes such as maleimide, hydroxyls, sulfonic acids, mercaptans, and the like. For example, coupling of analytes and other biological molecules to agarose and polyacrylamides is described by W.

B. Jacoby and M. Wilchek, *Methods in Enzymology*, Vol. 34, Academic Press, New York (1974).

The analyte or binding analog thereof immobilized to the magnetically responsive reagent particle as heretofore described can be selected for the determination of any analyte for which there is a binding counterpart available. The analyte usually is a peptide, polypeptide, protein, carbohydrate, glycoprotein, steroid, nucleic acid or other organic molecule for which a binding counterpart exists or which is producible in biological systems or can be synthesized. The analyte, in functional terms, is usually selected from the group comprising antigens, haptens, complementary polynucleotide sequences, hormones, vitamins, metabolites and pharamcological agents. Usually, the analyte is an immunologically-active polypeptide or protein, usually having a molecular weight of between about 1,000 and about 10,000,000, such as an antigenic polypeptide or protein, or a hapten having a molecular weight of at least about 100, and usually less than about 1,500.

Representative polypeptide analytes are angiotensin I and II, C-peptide, oxytocin, vasopressin, neurophysin, gastrin, secretin, bradykinin, and glucagon.

Representative protein analytes include the classes of protamines, mucoproteins, glycoproteins, globulins, albumins, scleroproteins, phosphoproteins, histones, lipoproteins, chromoproteins, and nucleoproteins. Examples of specific proteins are prealbumin, $\alpha_1$-lipoproteins, human serum albumin, $\alpha_1$-acid glycoprotein, $\alpha_1$-antitrypsin, $\alpha_1$-glycoprotein, transcortin, thyroxine binding globulin, haptoglobin, hemoglobin, glycosylated peptide sequences such as the glucosylated N-terminal peptide sequence in the beta-subunit of human hemoglobin, myoglobulin, ceruloplasmin, $\alpha_2$-macroglobulin, $\beta$-lipoprotein, erythropoietin, transferrin, hemopexin, fibrinogen, the immunoglobulins such as IgG, IgM, IgA, IgD, and IgE, and their fragments, e.g., $F_c$ and $F_{ab}$, complement factors, prolactin, blood clotting factors such as fibrinogen, thrombin and so forth, insulin, melanotropin, somatotropin, thyrotropin, follicle stimulating hormone, leutinizing hormone, gonadotropin, human chorionic gonadotropin, thyroid stimulating hormone, placental lactogen, instrinsic factor, transcobalamin, serum enzymes such as alkaline phosphatase, lactic dehydrogenase, amylase, lipase, phosphatases, cholinesterase, glutamic oxaloacetic transaminase, glutamic pyruvic transaminase, and uropepsin, endorphins, enkephalins, protamine, tissue antigens, bacterial antigens, viral antigens such as hepatitis associated antigens (e.g., $Hb_sAg$, $HB_cAg$ and $HB_eAg$), and tumor markers (e.g., CEA, alpha fetoprotein, prostatic acid phosphatase, prostatic specific antigen, neuron specific enolase, estrogen receptor. CA125, CA19-9, and the like).

Representative hapten analytes inlcude the general classes of drugs, metabolites, hormones, vitamins, toxins and the like organic compounds. Haptenic hormones include thyroxine and triiodothyronine. Vitamins include vitamins A, B, e.g., thiamine, B-2, C, D, E and K, and folic acid. Drugs include antibiotics such as aminoglycosides, e.g., gentamicin, tobramycin, amikacin, sisomicin, kanamycin, and netilmicin, penicillin, tetraycycline, terramycine, chloromycetin, and actinomycetin; nucleosides and nucleotides such as adenosine diphosphate (ADP) adenosine triphosphate (ATP), flavin mononucleotide (FMN), nicotinamide adenine dinucleotide (NAD) and its phosphate derivative (NADP), thymidine, guanosine and adenosine; prostaglandins; steroids such as the estrogens, e.g., estriol and estrafiol, sterogens, androgens, digoxin, digitoxigenin, digitoxin, digoxigenin, 12-0-acetyldiogoxigenin, and adrenocortical steroids; and others such as phenobarbital, phenytoin, primidone, ethosuximide, carbamazepine, valproate, theophylline, caffeine, propranolol, procainamide, quinidine, amitryptiline, cortisol, desipramine, disopyramide, doxepin, doxorubicin, nortryptiline, methotrexate, imipramine, lidocaine, procainamide, N-acetylprocainamide, amphetamines, catecholamines, and antihistamines. Toxins include acetyl T-2 toxin, alfatoxin, cholera toxin, citrinin, cytochalasins, staphylococcal enterotoxin B, HT-2 toxin, and the like.

Labeled Reagent

The labeled reagent employed according to the present invention comprises a specific binding partner for the analyte under determination labeled with a detectable chemical group. The specific binding partner is one which is capable of binding to either the analyte from the liquid test medium or to the analyte or binding analog thereof immobilized to the magnetically responsive reagent particle.

The specific binding partner for the analyte can be a whole antibody, such as any of the classes and subclasses of known immunoglobulins, e.g., IgG, IgM, and the like, or monovalent and divalent antibody fragments of IgG, conventionally known as Fab and Fab', and F(ab)₂, respectively. The immunoglobulin source for the antibody component can be obtained in any available manner such as conventional antiserum and monoclonal techniques. Antiserum can be obtained by well-established techniques involving immunization of an animal, such as a mouse, rabbit, guinea pig or goat, with an appropriate immunogen. The immunoglobulins can also be obtained by somatic cell hybridization techniques, such resulting in what are commonly referred to as monoclonal antibodies, also involving the use of an appropriate immunogen.

Preferably, the antibody will commonly be a divalent antibody fragment [F(ab')₂] or, more preferably, a monovalent antibody fragment (Fab or Fab'). Divalent and monovalent IgG antibody fragments can be obtained according to methods known in the art employing standard proteolytic digestion procedures with pepsin or papain. For example, divalent antibody fragments [F(ab')₂] can be prepared by digesting the whole IgG antibody with pepsin [Nisonoff, *Methods Med. Res.,* Vol. 10, 132(1964)], and then reducing the F(ab)₂ fragment to the corresponding Fab' fragment with dithiothreitol. Alternatively, Fab fragments can be obtained by papain digestion of IgG [Porter, *Biochem. J.,* Vol. 73, 119(1959)].

The detectable chemical group of the labeled reagent can be any material having a detectable physical or chemical property. Such materials have been well-developed in the field of immunoassays and in general any label useful in such methods can be applied to the present invention.

In particular, such chemical groups having detectable physical properties are those groups which are detected on the basis of their own physical properties which do not require a chemical reaction or interaction with another chemical or substance to provide a detectable signal. Such groups principally include fluorescers such as umbelliferone, fluorescein, resorufin, various rhodamines, dansyl derivatives and aminoanaphthalenesulfonic acid [see *Clin. Chem.,* 25:353(1979)]; phosphorescent molecules such as pyrene, 4-nitrobiophenyl, benzaldehyde, benzophenone or the trivalent metal chelates of dibenzoylmethane (e.g., $A1^{+3}$, $T^{+3}$); chromophores such as para- or ortho-nitrophenol, phenolphthalein, napthol AS, para-nitroanilide and thymolphthalein; radioisotopes such as $^3H$, $^{35}S$, $^{32}P$, $^{125}I$ and $^{14}C$; spin labels including nitroxide radicals such as DOXYL, PROXYL and TEMPO derivatives; or electroactive moieties such as protons, fluoride, oxygen, ammonia and hydrogen peroxide.

Chemical groups having detectable chemical properties are those groups which are detected on the basis of their own chemical reactivity or interaction with a detectant component therefor to provide a detectable signal. Such chemical groups having detectable chemical properties do not generate a detectable product or otherwise provide a detectable signal prior to interacting with such detectant component and include enzymatically active groups such as enzymes (see *Clin. Chem.,* 22:1232(1976), U.S. Reissue Pat. 31,006, and U.K. Pat. 2,019,308), enzyme substrates (see British Pat. Spec. 1,548,741), coenzymes (see U.S. Pat. Nos. 4,230,797 and 4,238,565), enzyme inhibitors and activators, chemiluminescent species, chemical catalysts, metal catalysts, members of enzyme channeling, fluorophor-quencher or energy transfer pairs (see U.S. Pat. Nos. 3,996,345; 4,174,384; 4,199,559, and 4,233,401), and specifically bindable ligands such as biotin or a hapten.

Where the label of the labeled reagent is a detectable chemical group having a detectable chemical property, the detectant component therefore is incorporated into the detection zone of the separation and detection test device of the present invention. Preferably, such detectable chemical group is an enzyme, whereby the particular substrate for the selected enzyme label is incorporated into the detection zone of the test device. Particular enzymes which can be used include, but are not intended to be limited to, $\beta$-$\underline{D}$-galactosidase, peroxidase, glucose oxidase, alkaline phosphatase, glucose-6-phosphate dehydrogenase, luciferase, pyruvate kinase, lactate dehydrogenase, galactose oxidase, tyrosinase, invertase, xanthanine oxidase, urease, uricase, alcohol oxidase, and the like.

Chromatographic Separation and Detection Test Device

The free species of the labeled reagent immobilized to the magnetically responsive reagent particle is magnetically separated from the bound species of the labeled regent on a chromatographic separation and detection test device comprising a solid, porous matrix which is capable of transporting aqueous liquids by capillary action. The test device is composed of a separation zone and a detection zone which is situated a predetermined distance away from and in liquid transport contact with the separation zone. As will be described in greater detail hereinafter, the separation zone and the detection zone can be composed of and located along the length of a single, continuous matrix material, or can be composed of physically distinct matrix materials in liquid flow contact.

In particular, the solid, porous matrix material is permeable to aqueous liquids and at least some components of a liquid test sample, e.g, antigens, haptens, antibodies, and the like, and, where the magnetic field is applied simultaneously with the application of the reaction mixture to the separation zone, can be permeable to the magnetically responsive reagent particle as well. It is to be understood that the liquid test medium containing the analyte under determination can be a naturally occurring or artificially formed liquid suspected to contain analyte, and is usually a biological fluid or a dilution thereof. Such biological fluids include serum, whole blood, plasma, urine, saliva, and amniotic and cerebrospinal fluids. Accordingly, in most instances, selection of an appropriate matrix material will also be one which is capable of removing cellular components such as red blood cells, white blood cells, platelets, and the like, from such biological fluids.

Preferably, the solid, porous matrix material is selected from various fibrous materials known in the art which are capable of transporting aqueous liquids by capillary action. More preferably, the matrix material should be capable of rapid capillarity in order to transport the bound species of the labeled reagent from the separation zone into the detection zone upon contact with the liquid reaction mixture. Such matrix materials include, but are not intended to be limited to, paper, glass fiber, porous plastic, scintered glass or plastic, cellulose particles, gels of agarose or other polymers, or woven materials such as cloth, nylon or other polymeric mesh material, or other fibrous materials, whether formed from natural materials or synthetic materials. For example, U.S. Patent No. 3,846,247 describes the use of felt and woven or matted glass fibers, and the use of synthetic resin fleeces and glass fiber felts is described in Great Britain Patent No. 1,369,139. Other fibrous matrix materials are also described in U.S. Patent Nos. 3,802,842, 3,809,605, 3,897,214, and 3,987,214.

The various matrix materials employed according to the present invention can also be treated with substances known in the art to facilitate the permeability of the bound species of the labeled reagent. For example, detergents such as Tween 20®, Triton X-100®, or sodiumdodecylsulfate can be added to either the matrix materials or to the liquid test sample or reaction mixture. Similarly, protein such as bovine serum albumin, lactalbumin, or gamma globulin, can be added to the liquid test sample, the test device, or to the reaction mixture.

As will be understood by one skilled in the art, the magnetic separation of the free species of the labeled reagent immobilized to the magnetically responsive reagent particle permits the selection of a matrix material having optimal capillarity for the liquid transport of the bound species without having to consider, as a parameter for making such selection, the effective pore size of the matrix material. For example, where a matrix material is selected having optimal capillarity as heretofore described, but which comprises a minimum effective pore size or shape which is larger than the size of the magnetically responsive reagent particle and therefore permeable thereto, the magnetically responsive reagent particle will nevertheless be prevented from migrating out of the separation zone as a result of the magnetic field applied thereto. Similarly, where an optimal matrix material is selected having a minimum pore size which is substantially smaller than the size of the magnetically responsive reagent particle and therefore impermeable thereto, the magnetically responsive reagent particle will nevertheless be prevented from migrating across the surface and out of the separation zone, and onto the surface of the detection zone, as a result of the magnetic field applied thereto. Accordingly, selection of an appropriate matrix material can be made by one skilled in the art apprised of the foregoing considerations.

Where the separation zone and the detection zone are composed of a single, continuous matrix material as heretofore described, the detection zone will be, of course, composed of the same matrix material and possess the same capillarity as the separation zone matrix material. It will be understood, however, that where it is desirable for the separation zone and the detection zone to be composed of physically distinct matrix materials, such materials can be the same or different, whereby the detection zone matrix material can be selected from the aforementioned matrix materials. Where such matrix materials are different, the detection zone matrix material must, of course, at least be capable of transporting aqueous liquids by capillary action.

According to the present invention, the detection zone receives the bound species of the labeled reagent which migrates out of the separation zone and into the detection zone by capillary action. Once in the detection zone, the detectable signal provided by the label of the bound species of the labeled reagent is measured and correlated to the amount of analyte present in the liquid test sample. The generation of the detectable signal will of course depend upon the particular label employed, i.e., that which possesses either a detectable physical property or a detectable chemical property. For example, where the label possesses a detectable physical property, the label of the bound species inherently provides a detectable signal which, once in the detection zone, can be readily measured without interacting with a detectant component therefore to provide the detectable signal in the detection zone. On the other hand, where the label possesses a detectable chemical property, the detection zone is incorporated with the detectant component for the label, such as an enzyme substrate where the label is an enzyme, which interacts with the label to provide the detectable signal in the detection zone. In either case, the signal generated by the label of the bound species in the detection zone is measured with an appropriate instrument known in the art, such as a spectrophotometer, particularly a Seralyzer® reflectance photometer (Miles Diagnostics, Elkhart, IN, USA) with which the test device is readily adaptable.

According to one preferred embodiment of the present invention (Fig. 1), the separation zone and the detection zone are located at physically distinct portions along the length of an elongate strip composed of a single, continuous matrix material. The separation zone and detection zone are situated a predetermined distance away from and in liquid transport contact with each other along the length of the strip. A source for applying a magnetic field to the separation zone, such as a permanent magnet or an electromagnet, is positioned under the separation zone for the magnetic separation of the magnetically responsive reagent particle at one end of the strip, and the detection of the bound species of the labeled reagent at the other end of the strip, respectively.

In particular, an aliquot of the reaction mixture is applied to the separation zone and the magnetic field applied thereto whereby the magnetically responsive reagent particle is retained at the separation zone by the magnet field and thereby prevented from migrating into the detection zone, and the bound species of the labeled reagent is transported by capillary action into the detection zone. Depending upon the effective pore size of the matrix material as previously described, the magnetic field can be applied to the separation zone prior to, simultaneously with, or subsequent to, the application of the reaction mixture. Preferably, and as a matter of convenience, the magnetic source is first positioned under at least the separation zone and the reaction mixture is then applied to the separation zone. Where an instrument such as a Seralyzer® reflectance photometer is employed for the detection and measurement of the detectable signal provided by the bound species of the labeled reagent in the detection zone, the test strip table or holder thereof can be incorporated with a permanent magnet or an electromagnet over which at least the separation zone can be positioned. As will be understood by one skilled in the art, the magnetic source should provide a magnetic field which is of a sufficient strength to retain the paramagnetic reagent particles at the site of application thereof onto the separation zone. Such strength will, of course, depend upon such parameters as the size and density of the paramagnetic material of the reagent particles and the distance of the magnetic source from the surface of the test device, and will generally be at least 400 Gauss. Furthermore, although the magnetic source should be positioned at least under the site of application of the paramagnetic reagent particles onto the separation zone, the dimensions of the magnetic source can be larger than the dimensions of the separation zone and can extend into the area of the detection zone as well.

It is to be understood that since the separation of the magnetically responsive reagent particle from the bound species of the labeled reagent arises from the ability of the matrix material to transport aqueous liquids by capillary action, such separation is therefore best achieved by transporting a relatively large volume of the liquid test medium through a relatively narrow area. Accordingly, the width of the elongate strip is generally, but not intended to be limited to, less than 20 mm wide, preferably from between about 1.0 mm and 23 mm wide, more preferably from between about 4.0 mm to 8.0 mm wide. The length of the strip is generally, but not intended to be limtied to, from between about 2.0 cm and 40 cm long, preferably from between about 4.0 cm to 25 cm long, more preferably from between about 6.0 cm to 20 cm long.

According to another preferred embodiment of the present invention (Fig. 2), the separation zone and the detection zone are composed of, individually, physically distinct portions similarly located along the length of an elongate strip and composed of physically distinct matrix materials. In particular, the individual matrix materials, which can be the same or different as heretofore described, are positioned in an end-to-end relationship whereby the ends thereof are in liquid transport contact to permit the migration of the bound species of the labeled reagent into the detection zone. A source for applying a magnetic field is similary positioned under the separation zone in order to effect the magnetic separation of the magnetically responsive reagent particle as heretofore described. It is to be understood that although the individual matrix materials preferably form an elongate strip having dimensions similar to those described above, the individual matrix materials forming the separation zone and the detection zone can vary in length whereby the length of one can be different from, and not necessarily the same as, the length of the other.

The thickness of the separation zone and detection zone matrix materials as heretofore described, as well as the degree of permeability thereof, will vary and depend upon actual usage. Generally, a dry thickness from between about 0.1 mm and 2.0 mm is preferable, although more widely varying thicknesses can be selected by one skilled in the art apprised of the foregoing considerations and depending upon the particular circumstances of usage. Furthermore, the separation zone and detection zone matrix materials are preferably mounted or otherwise positioned onto a solid, nonporous support member with an appropriate adhesive. A wide variety of organic and inorganic materials, both natural and synthetic, and combinations thereof, may be employed provided only that the support does not interfere with the capillary action of the strip, or non-specifically bind assay components, or interfere with the signal producing system. Illustrative polymers include polyethylene, polypropylene, poly(4-methylbutene), polystyrene, polymethacrylate, poly(ethylene terephthalate), nylon, poly(vinyl butyrate), glass, ceramics, metals, and the like.

## Test Kit

The present invention further provides a test kit comprising all of the essential elements required to conduct the immunoassay method of the present invention. The test kit is presented in a commercially packaged form, as a composition or admixture where the compatability of the reagents will allow, in a test device configuration, or more usually as a test kit, i.e., a packaged combination or one or more containers, devices or the like holding the necessary reagents, and usually including written instructions for the performance of the immunoassay.

In particular, the test kit will at least comprise (1) magnetically responsive reagent particles having the analyte or a binding analog thereof immobilized thereto, preferably a uniform latex suspension thereof, (2) a labeled reagent, comprising a labeled form of a specific binding partner for the analyte, preferably a monovalent antibody fragment derived from a monoclonal antibody to the analyte under determination, labeled with an enzyme, and (3) a chromatographic separation and detection test device for separating the magnetically responsive reagent particles, preferably incorporated with a substrate for the enzyme, which generates a detectable signal which can be measured and correlated to the amount of analyte present in a liquid test sample.

In particular, the present invention will now be illustrated, but is not intended to be limited, by the following examples:

### Example 1

#### Separation of Paramagnetic Latex Particles on a Paper Matrix Material

In order to demonstrate the magnetic separation of paramagnetic latex particles on a paper matrix material, a sheet of Whatman 3 mm paper (Whatman, Ltd., Maidstone, England) was cut into 0.7 cm x 2.0 cm elongate test strips, and one end of each of the strips identified as the separation zone and the other end thereof identified as the detection zone.

(i) A permanent magnet (Edmund Scientific, Barrington, N.J., USA, rectangular 8500 Gauss magnet cat. no. H30,964) was positioned approximately 1.6 cm beneath a test strip (at least 400 Gauss at the surface of the test strip) and a 30 $\mu$l aliquot of a 1.0% (weight/volume) aqueous suspension of paramagnetic ferric oxide (95%) latex particles (Magic™ particles for TSH assay, mean diameter from between about 0.5 and 1.5 $\mu$m, Corning Medical, Medfield, MA, USA) in 10% human serum was pipetted onto a centrally located area of the test strip at approximately 3.2 cm from the end of the separation zone. A 30 $\mu$l aliquot of the aforementioned paramagnetic latex particle suspension was similarly pipetted onto another test strip without a permanent magnet positioned thereunder. In the presence of the magnetic field, the particles were visually observed (brown coloration) only at the site of application thereof on the test strip, whereas in the absence of the magnetic field, the particles were visually observed in both the separation zone and the detection zone.

(ii) A permanent magnet was positioned under four test strips as described above, and 30 $\mu$l aliquots of 0.5% and 1.0% (weight/volume) aqueous suspensions of paramagnetic ferric oxide (12%) latex particles (1.0 $\mu$m diameter, Seragen Diagnostics, Inc., Indianapolis, IN, USA) in 10% human serum, and 0.5 % and 1.0% (weight/volume) suspensions of the paramagnetic latex particles in 100% human serum, were similarly pipetted onto each of the test strips, respectively, as described above. 30 $\mu$l aliquots of the aforementioned paramagnetic latex particle suspensions were similarly pipetted onto four test strips, respectively, without a permanent magnet positioned thereunder. In the presence of the magnetic field, the particles were visually observed (brown coloration) only at the site of application thereof on each of the test strips, whereas in the absence of the magnetic field, the particles were visually observed in both the separation zones and the detection zones of the test strips.

## Example 2

Preparation of Chromatographic Separation and Detection Test Device and Use Thereof in an Immunoassay

### (a) Test Device

A 3.81 cm x 15.24 cm sheet of Whatman 54 (Whatman, Ltd., Maidstone, England) is impregnated by immersion into 5.0 mM $MgCl_2$ in 0.1 M bicine (pH 8.3) and dried by forced air at 50% for 10 minutes. The sheet is then impregnated by immersion into 10.0 mM resorufin-galactoside (prepared according to the method described by European Patent Application Publication No. 156,347) in dimethylformamide, and dried by forced air at 50°C for 10 minutes. Double-faced adhesive tape is applied to one side of the impregnated glass fiber sheet, and then cut into 2.0 cm wide elongate strips. The elongate strips are than laminated at one end of 8.3 cm x 2.0 cm polystyrene sheets (0.4 cm thick), and then cut into 8.3 cm 0.5 cm elongate strips. The elongate strips are stored in brown glass bottles with a desiccant at room temperature.

### (b) Reagent Particles

3-Hydroxy-(5-carboxypentyl)-carbamoyl digitoxigenin (0.132 mmole) is added to 0.132 mmole isobutylchloroformate and 0.132 mmole triethylamine in 5.0 mL dimethylformamide at -10°C to -20°C under argon, and then added dropwise to a solution of bovine serum albumin (110.0 mg/10.0 mL) in 0.1 L sodium pyrophosphate (pH 8.5) with vigorous stirring at 0-4°C for a period of 15 minutes. The reaction mixture is then sonicated for one hour. After storage at 4°C overnight, the reaction mixture is adjusted to pH 7.0, loaded onto a 45.0 cm x 2.5 cm Sephadex® column (Sigma, St. Louis, MO, USA) equilibrated with 25.0 mM sodium phosphate buffer (pH 7.0), and eluted with 25.0 mM sodium phosphate buffer (pH 7.0).

Carboxylate modified polystyrene paramagnetic latex particles (1.3 $\mu$m average diameter, 0.02-0.05 mequiv. of carboxylate/g particles; Seragen, Inc., Indianapolis, IN, USA) are rinsed twice with water and resuspended at 5.0% (weight/volume) in 0.1 M borate buffer (pH 8.5) which contains 0.05 mg/mL of the BSA-digitoxigenin reagent described above. The suspension is incubated for 20 hours and washed three times with 20.0 mM sodium phosphate, 0.1% Tween 20 (pH 7.0), twice in 1.0% sodiumdodecylsulfate for 30 minutes at 60°C, once in 20.0 mM sodium phosphate, 0.1% Tween 20 (pH 7.0), and twice in ethanol. The paramagnetic latex particles are stored at 5.0% (weight/volume) in ethanol at 4°C. The ethanol is decanted and the paramagnetic latex particles resuspended to 1.0% (weight/volume) in reagent buffer (0.05 M sodium phosphate, 0.05 M NaCl, 1.0 mM MgCl$_2$, 0.02% NaN$_3$ and 0.01% bovine serum albumin, pH 7.4) before use.

(c) Immunoassay and Operation of the Test Device

An immunoassay reaction mixture is formed comprising digoxin calibrator serum (Optimate® liquid analyzer, Miles Diagnostics, Elkhart, IN, USA) diluted five-fold into the reagent buffer described above containing a labeled reagent (1.0 mM of a substantially pure anti-digoxin/$\beta$-D-galactosidase monoconjugate preparation comprising a single monovalent antibody fragment derived from an anti-digoxin monoclonal antibody coupled to a single $\beta$-D-galactosidase molecule). After 5 minutes at room temperature, an equal volume of 2.0% (weight/volume) of the paramagnetic latex reagent is added and incubated for 3 minutes at room temperature.

An elongate strip as described above is placed onto the strip holder of a Seralyzer® reflectance photometer (Miles Diagnostics, Elkhart, IN USA) having a permanent magnet (Crumax®, Colt Industries, Lexington, KY, USA) positioned thereunder, and 30.0 μl of the reaction mixture described above is applied to the separation zone of the elongate strip. The signal generated by the labeled reagent bound to the serum digoxin which migrates into the detection zone is measured [K/S rate signal response =

$$\frac{(1 - \text{reflectance})^2}{(2 \times \text{reflectance})},$$

determined between 70-90 seconds after placing the aliquot on the elongate strip].

The K/S rate response is linearly proportional to the original sample analyte concentration.

**Claims**

1. A method for the specific binding assay determination of an analyte in an aqueous liquid test medium employing (i) a magnetically responsive reagent particle comprising the analyte or a binding analog thereof immobilized thereto, (ii) a labeled reagent comprising a specific binding partner for the analyte labeled with a detectable chemical group, and (iii) a chromatographic separation and detection test device comprising a solid, porous matrix which is capable of transporting aqueous liquids and which comprises a separation zone and a detection zone which is situated a predetermined distance away from and in liquid transport contact with said separation zone, said method characterized by the steps of:

(a) forming an aqueous liquid reaction mixture comprising said test medium, said labeled reagent, and said magnetically responsive reagent particle in an amount in excess of that capable of binding to all of said labeled reagent which does not become bound to said analyte;

(b) applying said reaction mixture to said separation zone of said test device and applying a magnetic field to said separation zone whereby said magnetically responsive reagent particle is magnetically retained at said separation zone and said labeled reagent not bound to said magnetically responsive reagent particle is transported into said detection zone by said liquid medium; and

(c) measuring said detectable chemical group of said labeled reagent in said detection zone of said test device.

2. The method of claim 1 characterized in that said magnetically responsive reagent particle is a paramagnetic particle, preferably a water suspensible, paramagnetic latex particle.

3. The method of claim 1 or 2 characterized in that said aqueous liquid reaction mixture is formed by (i) forming a first reaction mixture comprising said test medium and said labeled reagent, said labeled reagent being present in said reaction mixture in an amount in excess of that capable of binding to all of said analyte suspected to be in said test medium; (ii) incubating said first reaction mixture for a predetermined period of time; (iii) forming a second reaction mixture comprising said first reaction mixture and said magnetically responsive reagent particle; and (iv) incubating said second reaction mixture for a predetermined period of time.

4. The method of any one of claims 1 to 3 characterized in that said test device comprises an elongate strip composed of a solid, porous matrix material capable of transporting aqueous liquids along the length thereof by capillary action, said separation and detection zones being located at physically distinct portions along said strip.

5. The method of claim 4 characterized in that said elongate strip is composed of a single, continuous matrix material, preferably a fibrous matrix material selected from paper, fiberglass, cellulose or a woven fabric.

6. The method of claim 4 or 5 characterized in that said physically distinct portions of said strip comprising said separation and detection zones are composed of physically distinct matrix materials in liquid transport contact.

7. The method of any one of claims 1 to 6 characterized in that said detectable chemical group possesses a detectable chemical property and interacts with a detectant component to provide a detectable signal.

8. The method of claim 7 characterized in that said detection zone is incorporated with said detectant component.

9. A test system for the specific binding assay determination of an analyte in an aqueous liquid test medium characterized in that said test system comprises:

(i) a chromatographic separation and detection test device comprising a solid, porous matrix which is capable of transporting aqueous liquids and which comprises a separation zone and a detection zone which is situated a predetermined distance away from and in liquid transport contact with said separation zone, and to the separation zone which has been applied a reaction mixture comprising said liquid test medium, a labeled reagent comprising the analyte or a binding analog thereof labeled with a detectable chemical group, and a magnetically responsive reagent particle comprising the analyte or a binding analog thereof immobilized thereto and in an amount in excess of that capable of binding to all of said labeled reagent which does not become bound to the analyte, and

(ii) means for applying a magnetic field to magnetically retain said magnetically responsive reagent particle at said separation zone,

whereby said labeled reagent bound to the analyte is transported into said detection zone by said liquid test medium and is detectable therein as a function of the amount of analyte present in said liquid test medium.

10. A test kit for the specific binding assay determination of an analyte in a liquid test medium characterized in that said test kit comprises

(a) a magnetically responsive reagent particle comprising the analyte or a binding analog thereof immobilized thereto;

(b) a labeled reagent comprising a specific binding partner for the analyte labeled with a detectable chemical group; and

(c) a chromatographic separation and detection test device comprising a solid, porous matrix which is capable of transporting aqueous liquids and which comprises a separation zone and a detection zone which is situated a predetermined distance away from and in liquid transport contact with said separation zone.

SUPPORT MEMBER

SEPARATION ZONE

DETECTION ZONE

MAGNET

FIG. 1

SUPPORT MEMBER

SEPARATION ZONE

DETECTION ZONE

MAGNET

FIG. 2